(19) **Europäisches Patentamt**

**European Patent Office**

**Office européen des brevets**

(11) Publication number: **0 410 750 B1**

(12) **EUROPEAN PATENT SPECIFICATION**

(45) Date of publication of patent specification: **08.03.95**

(51) Int. Cl.⁶: **A61K 38/00**, A61K 38/21, C07K 1/00, C12P 21/02, //C12N15/24,(A61K38/21,38:00)

(21) Application number: **90308198.2**

(22) Date of filing: **26.07.90**

Consolidated with 90911066.0/0484380 (European application No./publication No.) by decision dated 24.09.92.

(54) **Use of Interleukin-4 for increasing numbers of neutrophils and for treating myeloid or monocytoid leukaemia.**

(30) Priority: **28.07.89 US 386937**

(43) Date of publication of application:
**30.01.91 Bulletin 91/05**

(45) Publication of the grant of the patent:
**08.03.95 Bulletin 95/10**

(84) Designated Contracting States:
**AT BE CH DE DK ES FR GB GR IT LI LU NL SE**

(73) Proprietor: **SCHERING CORPORATION**
**2000 Galloping Hill Road**
**Kenilworth**
**New Jersey 07033 (US)**

(72) Inventor: **Bonnem, Eric**
**622 Belvidere Avenue**
**Plainfield,**
**New Jersey 07062 (US)**
Inventor: **Sullivan, Lee**
**120 Roosevelt Boulevard**
**Edison,**
**New Jersey 08837 (US)**
Inventor: **Grace, Michael**

**22 Bonnie Rae Drive**
**Hamilton Township,**
**New Jersey 08620 (US)**
Inventor: **Bober, Loretta**
**301 Raritan Road**
**Linden,**
**New Jersey 07039 (US)**
Inventor: **Tang, John Chu-Tay**
**19 Camelot Drive**
**Livingston,**
**New Jersey 07039 (US)**
Inventor: **Naveh, David**
**P.O. Box No. 251**
**NL-2300 AG Leiden (NL)**
Inventor: **Nagabhushan, T. L.**
**3 Sunset Lane**
**Parsippany,**
**New Jersey 07054 (US)**
Inventor: **Raman, Jay**
**25 Highmont Drive**
**West Windsor,**
**New Jersey 08691 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

(74) Representative: **Ritter, Stephen David et al**
**Mathys & Squire**
**100 Grays Inn Road**
**London WC1X 8AL (GB)**

**Description**

This invention relates to the use of interleukin-4 (IL-4) in a method of increasing the numbers of neutrophils and in a method for treating myeloid or monocytoid leukaemia.

Interleukin-4 (IL-4) is a lymphokine (stimulator of the immune system) that has a broad range of immune-cell stimulatory activities [Banchereau et al., *Lymphokine Res.* Vol. 6, No. 1: U135 (1987); Yokoto et al., *Proc. Natl. Acad. Sci. USA,* 83: 5894-5898 (1986); Lee at al., *Proc. Natl. Acad. Sci. USA,* 83: 2061-2065 (1986); Coffman et al., *J. Immunol.* 136: 949-954 (1986); Sanderson et al., *Proc. Natl. Acad. Sci. USA,* 83: 437-440 (1986); Grabstein et al., *J. Exp. Med.,* 163: 1405-1413 (1985); and Vitetta et al., *J. Exp. Med.* 162: 1726-1731 (1985)]. At various times, IL-4 has also been referred to as B-cell growth factor (BCGF) [Butler et al., *J. Immunol.* 133: 251-255 (1984)(human BCGF); and Farrar et al., *J. Immunol.* 131: 1838-1842 (1983)(mouse BCGF)] and B-cell stimulatory factor 1 (BSF-1) [Ohara et a., *J. Immunol.* 135: 2518-2523 (1985)]. The name interleukin-4 was finally proposed and adopted in 1986 [Sanderson et a., *Proc. Natl. Acad. Sci. USA,* 83: 437-440 (1986)].

IL-4 was originally thought to be important only for the co-stimulation of activated B-cells [Roehm, et al., *J. Exp. Med.* 160:679-694 (1984)]. It has also been shown, however, to modulate the activities of T-cells and mast cells [Mosmann, et al., *Proc. Natl. Acad. Sci. USA.* 83:5654-5658 (1986)]. See also WO 87/0290, where the activity of IL-4 as a T-cell growth factor and B-cell growth factor is described as being useful to enhance natural defenses against various infections.

T-cells and B-cells act in the later stages of an immune response. It would be highly advantageous to have an agent which would increase and activate neutrophils which act in the early stages of infection and are the body's first line of defense against infection.

In the normal process of white blood cell hematopoiesis, cells originate in the bone marrow from a primitive immature cell known as a stem cell and differentiate through progressively more mature stages along different lineage pathways, to arrive at a terminal state of differentiation as a monocyte, granulocyte or lymphocyte.

A property of immature, undifferentiated cells is the ability to multiply rapidly. It is only when a precursor cell matures and differentiates through these multiple stages that it generally loses its capacity to proliferate and assumes the role of a specialized, functional mature cell. In the normal state, cells that reach their final mature form do not proliferate to any great extent, if at all.

In general, cancer is a disorder of cell differentiation. In particular, myeloid leukemias are disorders in which cells of monocytic and granulocytic lineages are blocked at an early stage of maturation and thus have not lost their proliferative capacity. Because these maturation-arrested cells continue to proliferate, they give rise to a population of immature cancer cells, resulting in a diagnosis of leukemia.

There is evidence that various myeloid leukemia cells can be induced to differentiate into normal macrophages and granulocytes and that upon differentiation these cells lose their capacity to proliferate. This suggests that the induction of terminal differentiation by an agent would be useful as a therapy for myeloid leukemia.

SUMMARY OF THE INVENTION

We have now surprisingly found that administration of IL-4 increases the neutrophil count in mammals and stimulates differentiation of neutrophils and monocytes. Even more surprisingly, we have found that the effects on neutrophils continue long after dosing with IL-4 has been terminated. This is very surprising since the half-life of IL-4 in the body is very short. We have thus discovered that IL-4 may be administered to a mammal to increase the numbers of neutrophils and to provide increased host resistance to infection or to treat infection at a very early stage. The mammal may be an immunocompromised host, e.g., any host susceptible to unwanted bacterial infection such as a patient having severe burns or ulcers, a host whose immune defenses are lowered because of radiation or chemotherapy in the treatment of cancer, and a host with a genetic immunodeficiency. These properties of IL-4 also indicate that it would be useful in topical administration to heal wounds such as open cuts or burns, by stimulating neutrophil and monocyte activation and fibroblast proliferation at the wound site.

We have also discovered that IL-4 induces the maturation of myeloid and monocytoid cells. Since myeloid leukemia is associated with the proliferation of immature myeloid cells, IL-4 by progressing myeloid cells to their mature state should reduce their proliferation and provide a method for treating myeloid leukemia.

Preferably, the mammals will be treated with IL-4 derived from a human source, i.e., human IL-4 such as human IL-4 produced recombinantly from E. coli or CHO cells. Most preferably, the dosage for the mammals will be administered by subcutaneous or intravenous injection or by intravenous infusion and will be in an amount of 0.1 to 30 micrograms of IL-4 per kilogram of body weight per day. Preferably, the IL-4 is administered in an amount of 1 to 15 micrograms of IL-4 per kilogram of body weight per day, and most preferably 3 to 10 micrograms of IL-4 per kilogram of body weight per day.

BRIEF DESCRIPTION OF THE DRAWINGS

Figure 1 illustrates the amino acid sequence of a preferred (human) IL-4 for use in the present invention.

Figure 2 is graphic representation of the increase in neutrophil cell count found by dosing cynomologus monkeys with IL-4.

Figure 3 is a graphical representation of the results indicating activation and differentiation achieved by treatment of HL-60 cells with IL-4.

Figure 4 is a graphical representation of the results indicating activation and differentiation achieved by treatment of U-937 cells with IL-4.

DESCRIPTION OF THE INVENTION

Any suitable IL-4 may be employed in the present invention. Complementary DNAs (cDNAs) for IL-4 have recently been cloned and sequenced by a number of laboratories, e.g., Yokoto et al., *Proc. Natl. Acad. Sci. USA*, 83: 5894-5898 (1986) (human); Lee at al., *Proc. Natl. Acad. Sci. USA,* 83: 2061-2065 (1986)(mouse); and Noma et al., *Nature* 319: 640-646 (1986)(mouse). Le et al., J. Biol. Chem. 263: 10817 (1988) have described the production of recombinant human IL-4 in CHO cells. IL-4 is also an article of commerce, available, e.g., from Genzyme Corporation, Boston, Massachusetts (human and mouse). Moreover, non-recombinant IL-4 has been purified from various culture supernatants, e.g., Sanderson et al., *Proc. Natl. Acad. Sci. USA,* 83: 437-440 (1986)(mouse); Grabstein et al., *J. Exp. Med.*, 163: 1405-1413 (1985)-(mouse); Ohara et al., J. Immunol., 135: 2518-2523 (1985)(mouse BSF-1); Butler et al., *J. Immunol.,* 133: 251-255 (1984)(human BCGF); and Farrar et al., *J. Immunol.,* 131: 1838-1842 (1983)(mouse BCGF).

Preferably, the IL-4 used in the present invention will be a human IL-4, and most preferably it will be the human version with the sequence described in Yokoto et al., *Proc. Natl. Acad. Sci. USA*, 83: 5894-5898 (1986) and PCT Patent Application No. 87/02990 published May 21, 1987.

According to this invention, mammals are administered an effective amount of an IL-4 to increase the numbers of monocytes and/or granulocytes (which might be any of all of the following: polymorphonuclear cells, eosinophils and/or basophils). Such an effective amount is defined as any amount of IL-4 that will significantly increase the neutrophil count, with an increased count of at least 25 percent, preferably 50%, considered significant. From 0.1 to 30 micrograms of IL-4, preferably human IL-4 (hIL-4), per kilogram of body weight per day is preferably administered. More preferably, mammals are administered 1.0 to 15.0 micrograms of hIL-4 per kilogram of body weight per day, and most preferably mammals are administered 3.0 to 10.0 micrograms of hIL-4 per kilogram of body weight per day.

The amount, frequency and period of administration will vary depending upon factors such as the level of the neutrophil and monocyte count (e.g., the severity of the monocytopenia or granulocytopenia), age of the patient, nutrition, etc. Usually, the administration will be daily initially and it may continue periodically during the patient's lifetime. Dosage amount and frequency may be determined during initial screenings of neutrophil count and the magnitude of the effect of IL-4 upon the increase in neutrophil count. Dosage will be aimed at increasing the neutrophil count to an acceptable level of about 1000 total neutrophils and/or 100 total monocytes to generate the desired biological effect, which will need to be determined individually for each patient depending on clinical circumstance. Additionally, selective manipulations may be performed, such as the enhancement of one or more subpopulations of lymphocytes.

To complement the neutrophil increasing effect of the IL-4, it may be useful to administer it in conjunction with other biologically and/or pharmaceutically active compounds. For example, it can be combined with other white-cell increasing agents [e.g., granulocyte-macrophage colony stimulating factor (GM-CSF) and granulocyte-colony stimulating factor (G-CSF)]. It also might be useful to combine IL-4 with other interleukins, e.g., with IL-1 and/or IL-3 and/or IL-7, for the purposes of increasing the total white blood cell and neutrophil count. IL-2 in combination with IL-4 may produce selective enhancement of specific and useful T-cell functions, and IL-4 in combination with IL-5 and/or IL-6 may be useful in specifically enhancing the function and/or numbers of normal and/or neoplastic B-cells. IL-4 may also be useful in increasing the utility of chemotherapeutic agents, including but not limited to alkylating agents, mitotic spindle poisons or antitumor antibiotics. By enhancing the number of white cells or the functional or differentiational status of specific subpopulations of cells, the efficacy of the aforementioned chemotherapeutic agents may be enhanced. The combination of an interferon, e.g., interferon gamma or alpha, with IL-4 may also be useful in increasing numbers and functions of certain white cell, particularly T-cell, subsets. In certain situations, to achieve the necessary biological effect, antibodies to any of the aforementioned interleukins or interferons may be administered instead of the native molecules.

Administration of the dose can be by the intravenous, nasal, parenteral, oral, subcutaneous, intramuscular, topical or transdermal route or by any other acceptable route. The IL-4 can be administered in any number of conventional dosage forms. Parenteral preparations include sterile solutions or suspensions. Inhalation administration can be carried out in the form of a nasal or oral spray, or by insufflation. Topical dosage forms can be creams, ointments, lotions, transdermal devices (e.g., of the conventional reservoir or matrix patch type) and the like.

The formulations and pharmaceutical compositions contemplated by the above dosage forms can be prepared with conventional pharmaceutically acceptable excipients and additives, using conventional techniques.

Presently, the IL-4 is preferably administered via the intravenous route. The solutions to be administered may be reconstituted lyophilized powders and they may additionally contain preservatives, buffers, dispersants, etc.

Preferably, IL-4 is reconstituted with 10 millimolar citrate buffer and preservative-free sterile water with the maximum concentration not to exceed 100 micrograms per milliliter and administered by continuous intravenous infusion or by intravenous injection. For continuous infusion, the daily dose can be added to 5 ml of normal saline and the solution infused by mechanical pump or by gravity.

The effect of IL-4 on increasing the neutrophil count in mammals can be determined by the following test protocol.

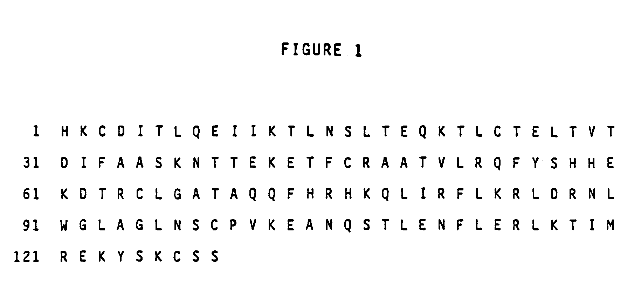

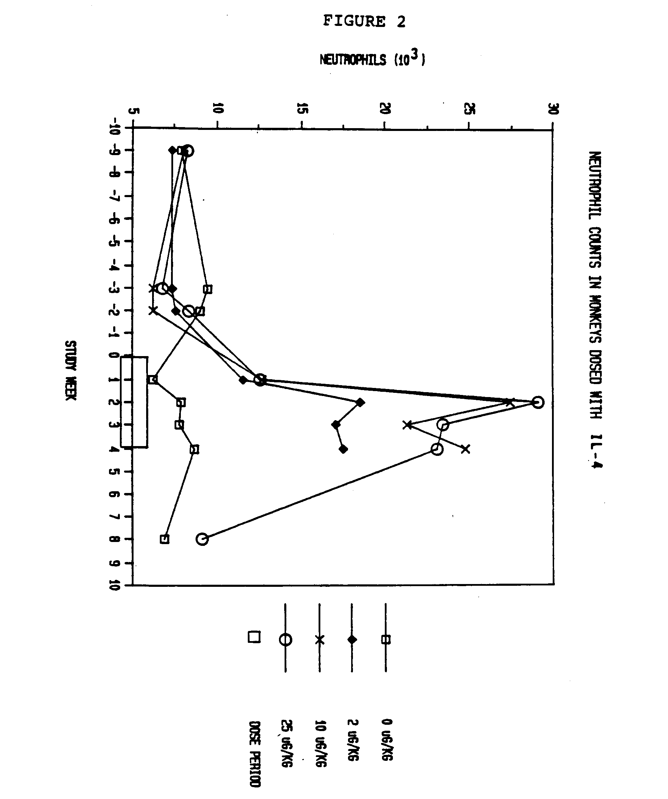

E. coli-derived human IL-4 having the amino acid sequence set forth in Figure 1 was evaluated in a one month study in cynomolgus monkeys. IL-4 was administered intravenously once daily at doses of 2, 10 and 25 μg/kg/day. Hematological and clinical chemical evaluations of blood samples from the monkeys were conducted at selected time points prior to, during, and one month after termination of dosing. Data were derived from a Coulter S + 4 hemotology analyzer [total white count] and a manual differential count. The results, shown in Figure 2, demonstrate the increased neutrophil count produced by the present invention.

The activation of neutrophils by IL-4 can be demonstrated by the following test protocols.

METHODS

A. Isolation of cells: Whole blood drawn from cynomolgus monkeys four weeks after the cessation of IL-4 dosing (25 μg/kg) or vehicle was subjected to gravity sedimentation through 6% dextran, followed by alternate lysis with hypotonic saline or Tris-amonium chloride to recover leukocytes.

B. Yeast Phagocytosis Assay: The phagocytic assay was performed by adding 300 μl of human serum, 50 μl of heat-killed yeast particles ($10^8$ organisms/ml) to 12 x 75 mm polypropylene tubes containing approximately $5 \times 10^5$ of the isolated leukocyte cells. Incubation was carried out for 90 minutes in a gyrorotary water bath at 37°C. Following centrifugation, the cell suspension was stained with 0.4% trypan blue and 0.2% eosin Y in saline. Ingested yeast remained colorless and uningested yeast stained purple, allowing for accurate determination of avidity (number of yeast cells ingested) as well as the percent of phagocytic cells. Mean values obtained for these parameters were multiplied to calculate a phagocytic index for each monkey. Data were analyzed by the Student's T test, comparing values from IL-4-treated monkeys to those of control monkeys receiving vehicle alone. The results are shown in Table 1.

5

Table 1.   The Phagocytic Function of Peripheral Blood Leukocytes
Drawn from Monkeys Four Weeks After IL-4 Withdrawal

| Monkey # | IL-4[a] (µg/kg) | Yeast/cell[b] | Percent[c] Phagocytic | Phagocytic Index[d] |
|---|---|---|---|---|
| 1 | 0 | 3.8 ± 0.2 | 35.0 | 130.6 |
| 2 | 0 | 3.8 ± 0.2 | 50.0 | 186.5 |
| 3 | 0 | 5.3 ± 0.2 | 45.6 | 242.1 |
| 4 | 0 | 2.6 + 0.2 | 33.8 | 87.5 |
| | mean[e] | 3.9 ± 0.6 | 41.1 ± 4 | 161.6 ± 33.6 |
| 5 | 25 | 4.84 ± 0.2 | 82.5 | 399.3 |
| 6 | 25 | 4.8 ± 0.2 | 70.0 | 333.2 |
| 7 | 25 | 4.7 ± 0.2 | 71.4 | 336.4 |
| 8 | 25 | 3.8 + 0.2 | 61.9 | 232.6 |
| | mean[e] | 4.5 ± 0.3 | 71.5 ± 4[f] | 325.4 ± 34.5[g] |

[a]Cells obtained from monkeys dosed for 4 weeks with
IL-4 or vehicle followed by a 4-week washout period.

bMean yeast per cell = # yeast ingested/60 cells from each monkey; Mean $\pm$ standard error of the mean (SEM) based on cells counted.

cPercent Phagocytic cells = % of cells ingesting yeast/total cells x 100.

dPhagocytic Index = mean yeast/cell x % phagocytic cells.

eMean $\pm$ SEM of the individual values for the monkeys.

fP<.002, Student's T test, control vs. IL-4.

gP<.014, Student's T test, control vs. IL-4.

C. NBT Assay: Following isolation as above, leukocytes from cynomolgus monkeys were resuspended in 0.2 ml of RPMI-1640 containing 2% heat-inactivated fetal bovine serum and distributed into 12 x 75 mm polypropylene test tubes. To each tube, 0.1 ml of a 2 mg/ml solution of nitroblue tetrazolium (NBT) in 2% RPMI and 0.1 ml of a 0.25 $\mu$M stock of freshly prepared phorbol 12-myristate 13-acetate (PMA) in 2% RPMI were added to the cells. The cell suspension was incubated in a 37°C water bath for 30 minutes. After incubation, the tubes were centrifuged and supernatants were removed from the cell pellet. Cell pellets were dried for 1 hour at 37°C before extraction with N,N-dimethylformamide (DMF). One ml of DMF was added to the cell pellet, vortexed, and immediately incubated at 85°C for 20 minutes. The tubes were centrifuged and the colored DMF was collected for spectrophotometric analysis at 560 nm against a DMF blank. All measurements were performed within 30 minutes of termination of the incubation.

Calculation of reduced NBT (NBF; $\mu$g/ml) was done from a standard curve prepared by using serial dilutions of a 2 mg/ml NBT stock solution spotted on Whatman filter strips and dried. For the standard curve, NBT was reduced by exposure to 1 mM ascorbate solution in 0.2 N NaOH for 20 min. at room temperature in the dark. The filters were extracted with DMF and read at 560 nm. Calculation of the NBF/cell was obtained by dividing NBF, $\mu$g/ml, by the number of cells per ml of sample. The results are shown in Table 2.

Table 2. The Phagocytic Function of Peripheral Blood Leukocytes Drawn from Monkeys Four Weeks after IL-4 Withdrawal Measured by NBT Reduction.

| Monkey # | IL-4 Dosage[a] (µg/kg) | Absorbance (560 nm) | NBF/ml (µg/ml) | NBF[b] pg/cell |
|---|---|---|---|---|
| 1 | 0 | 0.095 | 3.9 | 0.3 |
| 2 | 0 | 0.075 | 3.1 | 0.3 |
| 3 | 0 | 0.113 | 4.5 | 0.7 |
| 4 | 0 | 0.033 | 1.5 | 0.3 |
| | | | mean | 0.4 ± 0.1[c] |
| 5 | 25 | 0.213 | 8.3 | 1.1 |
| 6 | 25 | 0.316 | 12.1 | 0.7 |
| 7 | 25 | 0.483 | 18.5 | 1.0 |
| 8 | 25 | 0.163 | 6.5 | 0.7 |
| | | | mean | 0.9 ± 0.1[c.d] |

[a]Cells obtained from monkeys dosed for 4 weeks with IL-4 or vehicle followed by a 4-week washout period.

[b]NBF, pg per cell, calculated from NBF µg/ml/total cells in sample. Cell number obtained by hemacytometer count using Turks solution prior to assay.

[c]mean ± SEM of the individual values for the monkeys.

[d]$P < 0.02$, Student's T Test, control vs. IL-4.

The above results demonstrate that phagocytic index of leukocytes obtained from IL-4-treated monkeys four weeks post-dosing was increased significantly over leukocytes obtained from control animals. This elevation in the phagocytic index was due to the increase in the percentage of cells ingesting yeast. There was a slight increase in the avidity of the cells (number of yeast ingested per cell) from the IL-4 post-dosage group. The NBT dye reduction response measured as pg/cell of NBF was increased in cells taken form IL-4 treated monkeys as compared to cells from monkeys in the vehicle-control group.

These two assays, yeast cell ingestion and NBT dye reduction, are measures of phagocytosis and the metabolic changes (respiratory burst) that are important stages in the sequence of events that lead to the destruction of infectious agents. The increases obtained in both parameters indicate that IL-4 can increase or amplify the phagocytic response.

U937 is a cell line (ATCC CRL 1593) established from malignant cells from a patient with diffuse histiocytic leukemia [Sunstrom et al., *Int. J. Cancer* 17: 565-577 (1976)]. This cell line exhibits characteristics that indicate it is an immature monocyte.

HL-60 is a cell line (ATCC CCL 240) established from a patient with acute pro myelocytic leukemia. These cells exhibit characteristics that indicate it is an immature neutrophil.

IL-4 is able to induce differentiation of these 2 immature cells as defined by an increase in phagocytic function, an increase in activation of the hexase monophosphate shunt (NBT reduction) which is a metabolic necessity for phagocytosis, an increase in the percentage of cells able to take up a stain specific for mature neutrophils (nahthol chloroacetate esterase) or mature monocytes (alpha napthyl acetate esterase) and an increase in cells positive for surface markers as demonstrated by FACS analysis for mature monocytes or mature neutrophils.

The effect of IL-4 on maturation of myeloid cells may be demonstrated by the following test procedures.

NBI (Nitroblue Tetrazolium) Reduction To Formazan

Measurement of Hexose Monophosphate Shunt Activation Associated with Respiratory Burst.
Muller, et al., *Agents & Actions* 11:384 (1981).
Baehner, et al., *New Eng. J. Med.* 278:971 (1968).
Salin and McCord, *J. Clin. Invest.* 54:1005 (1974).
Standardized for use with U-937 and HL-60 cell lines
1. Harvest target cells sufficient for 1E6 cells/well assay. Spin down on Sorvall at 1200 rpm 5 min. Wash with PBS and respin.
2. Bring up cells in sufficient RPMI + 2% FBS (2%) to aliquot 0.2 ml/well assay.
3. Aliquot cells at 0.2 ml/well in 24-well flat-bottom plate.
4. Add 0.1 ml of 20 $\mu$M PMA in 2% (to each well).
5. Add 0.1 ml of 2 mg/ml NBT in 2% (to each well).
6. Incubate plate at 37°C for 30 min.
7. Immediately remove 0.1 ml of cells and spin down at 200 rpm (low acceleration) for 5 min. using Shandon Cytospin.
8. Counterstain with Dif-Quick system and mount coverslip for counting.

The yeast phagocytosis assay as described above was also performed for both cell lines using the same methodology.

Naphthol AS-D Chloroacetate Esterase Stain For PMNS

Yam, et al., *Am. J. Clin. Path.* 55:283 (1971).
Li, et al., *J. Histiochem. Cytochem.* 21:1 (1973).
Standardized for staining HL-60 cell line.
1. Spin down 1-5E5 cells onto microscope slide at 200 rpm (low acceleration) 5 min. using Shandon Cytospin.
2. Fix slide for 2 min. in Citrate-Acetone-MeOH fixative at room temperature (R.T.).
3. Wash in deionizer (d.i.) water and air-dry 20 min.
4. Stain slides in AS-D stain for 30 min. at 37°C in dark.
5. Wash 3X in d.i. water.
6. Counterstain in acid-hematoxylin stain 5 min.
7. Wash 3X in d.i. water, air-dry, mount with coverslip.

Citrate-Acetone-MeOH Fixative:

Dilute Citrate concentrate 1:9 with d.i. water.
Add 18 ml Citrate solution, 27 ml Acetone, and 5 ml abs. MeOH (methanol).
Store at room temperature.
Prepare daily.

AS-D Stain:

Dilute Trizmal 6.3 1:9 with d.i. water.
Warm 50 ml dilute Trizmal 6.3 to 37°C and add with constant stirring contents of 1 capsule of Fast Corinth V salt.
When salt is completely dissolved add 2 ml of Naphthol AS-D Chloroacetate solution. The solution will appear quite turbid. Continue stirring 15-30 min. and add to coplin jar. Do not filter.

AS-D Solution:

Dissolve 1 capsule of Naphthol AS-D Chloroacetate (20 mg) in 2 ml of dimethyl formamide. Prepare immediately before use.
Use Sigma kit 90 - Naphthol AS-D Chloroacetate.

Alpha Naphthyl Acetate Esterase Stain For Macrophages

Yam, et al., *Am. J. Clin. Path.* 55:283 (1971).
Li, et al., *J. Histiochem. Cytochem.* 21:1 (1973).
Standardized for staining U-937 cell line.
1. Spin down 1-5E5 cells onto microscope slide at 200 rpm (low acceleration) 5 min. using Shandon Cytospin.
2. Fix slide in Citrate-Acetone-MeOH Fixative 30 min. at R.T.
3. Wash in distilled/deionized water and air-dry 20 min.
4. Stain slides in NE stain at 37°C for 30 min. in dark.
5. Wash 3X in distilled/deionized water.
6. Counterstain in Mayer's Hematoxylin 5 min. at R.T.
7. Wash in distilled/deionized water, air-dry, mount with coverslip.

Citrate-Acetone-MeOH Fixative:

Dilute Citrate concentrate 1:9 with distilled/deionized water.
Add 18 ml Citrate solution, 27 ml Acetone, and 5 ml abs. MeOH.
Store at room temperature.
Prepare daily.

NE Stain:

Dilute Trizmal 7.6 (mono[tris(hydroxymethyl)aminomethane] maleate; pH 7.6) 1:9 in distilled/deionized water. Warm dilute Trizmal 7.6 to 37°C and add with constant stirring contents of 1 capsule Fast Blue RR salt (4-Benzoylamine-2,5-dimethoxybenzene-diazonium chloride hemi[zinc chloride] salt). When salt is completely dissolved add 2 ml of NE solution. The solution will be yellow and slightly turbid. Continue stirring for 15-20 min. and add to coplin jar. Do not filter.

NE Solution:

Dissolve 1 capsule (20 mg) of alpha naphthyl acetate to 2 ml of ethylene glycol monomethyl ether. Prepare immediately before use.
Use Sigma kit 90 - alpha naphthyl acetate.
The results from the above procedures using HL-60 cells and U-937 cells are set forth below in Tables 3 and 4.

## Table 3.   The Effect of E. coli-derived IL-4 on the Function and Differentiation of HL-60 Cells.

| Treatment[a] | Yeast/Cell[b] | % Phagocytic Cells[c] | Phagocytic Index[d] | NBT[e] | CAE[f] |
|---|---|---|---|---|---|
| Media | 2.4 ± 0.3 | 18 ± 5 | 42 ± 12 | 17 ± 2 | 21 ± 5 |
| IL-4 | | | | | |
| 250 U/ml | 5.5 ± 1.4[g] | 42 ± 5[g] | 225 ± 45[g] | 33 ± 3 | 62 ± 3[g] |
| 25 U/ml | 5.1 ± 1.1[h] | 41 ± 4[g] | 207 ± 37[g] | 39 ± 4[g] | 56 ± 6[g] |
| 2.5 U/ml | 5.4 ± 1.5[g] | 36 ± 3[g] | 184 ± 38[g] | 35 ± 4[g] | 53 ± 3[g] |
| 0.25 U/ml | 3.7 ± 1.0 | 27 ± 5 | 96 ± 24[h] | 36 ± 4[g] | 40 ± 2[g] |
| 0.025 U/ml | 3.1 ± 0.6 | 25 ± .6 | 75 ± 18 | 23 ± 2 | 32 ± 7 |
| DMSO | 3.6 ± 0.4[g] | 71 ± 10[g] | 259 ± 54[g] | 92 ± 2[g] | 56 ± 2[g] |

a   HL-60 cells incubated for 6 days in media containing the designated concentrations of IL-4 or DMSO. Cultures fed with IL-4 or DMSO on day 3.

b   Mean ± SEM of 4 separate experiments; Mean yeast/cell = # yeast ingested/30 cells

c   Percent Phagocytic Cells = % of cells ingesting yeast/total cells x 100 Mean ± SEM of 4 separate experiments.

d   Phagocytic Index - Mean yeast/cell x % Phagocytic cells.

e   % of cells positive for NBT/100 cells; Mean ± SEM of 4 experiments.

f   % of cells positive for chloroacetate esterase enzyme activity/100 cells; mean of 4 experiments.

g   $P \leq .05$; Student T test, Media vs. IL-4 treated or DMSO treated groups.

h   $P \leq .10$; Student T test, Media vs. IL-4 treated or DMSO treated groups.

Table 4. The Effect of an _E. coli_-derived IL-4 on the Function and Differentiation of U937 cells.

| Treatment[a] | Yeast/Cell[b] | % Phagocytic Cells[c] | Phagocytic Index[d] | NBT[e] | a NE[f] |
|---|---|---|---|---|---|
| Media | 1.3 ± .04 | 10 ± 2 | 15 ± 3 | 5 ± 5 | 16 ± 4 |
| IL-4 | | | | | |
| 250 U/ml | 2.5 ± 2[g] | 25 ± 5[g] | 57 ± 15[g] | 38 ± 7[g] | 66 ± 7[g] |
| 25 U/ml | 2.4 ± .2[g] | 25 ± 5[g] | 63 ± 17[g] | 41 ± 1[g] | 64 ± 6[g] |
| 2.5 U/ml | 2.5 ± .2[g] | 20 ± 3[g] | 51 ± 10[g] | 33 ± 2[g] | 54 ± 7[g] |
| .25 U/ml | 1.9 ± .3[g] | 21 ± 5[h] | 43 ± 14[h] | 28 | 44 ± 4[g] |
| .025 U/ml | 1.7 ± .1[g] | 16 ± 4 | 28 ± 8 | 27 ± 2[g] | 29 ± 3 |

a U937 cells incubated for 6 days in media containing the designated concentrations of IL-4. Cultures fed on day 3.

b Mean yeast/cell = # yeast ingested/30 cells; Mean ± SEM of 4 experiments.

c % Phagocytic cells = # of cells ingesting yeast/Total cells x 100; Mean ± SEM of 4 experiments.

d Phagocytic Index = Mean yeast/cell x % Phagocytic cells; Mean ± SEM of 4 experiments.

e % of cells positive for NBT/100 cells; Mean ± SEM of 4 experiments.

f % of cells positive for a NE activity/100 cells; Mean ± SEM of 4 experiments.

g $P \leq .05$, Student T test; Media vs. IL-4 treated groups.

h $P \leq .10$ Student T test; Media vs. IL-4 treated groups.

Fluorescence-Activated Cell Sorter (FACS)

Analysis Of Monocyte And Neutrophil Surface Markers

HL-60 and U-937 cells were incubated in T-75 flasks at 37°C and 5% $CO_2$ with increasing levels of IL-4 (E. coli-derived recombinant human IL-4 (rhuIL-4), 8-ILE-1002) or controls; cells were split and fed on day 3. On days 1, 3, and 6 cells were removed, washed with RPMI 1640 and blocked with human heat-aggregated IgG to reduce potential Fc interference. After washing again with RPMI 1640, cells resuspended in 50 $\mu$l of diluted antibody (see following table for the monoclonal antibody panel) and incubated 30 minutes on ice. The cells were then washed in PBS, resuspended in 100 $\mu$l of diluted FITC-conjugated goat-anti-mouse IgG, or $IgG_{2b}$ and incubated for 30 minutes on ice. After the incubation of the second antibody, the cells were washed repeatedly in PBS, then resuspended in 1 ml PBS and run on a Becton-Dickinson FACScan for cytofluorometric analysis. Isotype controls for murine $IgG_{2b}$ and $IgG_1$ and second antibody controls were run for increase in positive cells above a constituative expression level (i.e., IL-4 induction of enhanced expression).

Monoclonal Antibody Panel:

| Mab | Ig Isotype | Cluster of Differentiation | Specificity |
|---|---|---|---|
| WEMG11 | $muIgG_1$ | UNK. | gp 110: Granulocytes |
| FMC 32 | $muIgG_1$ | UNK. | Myelomonocytes |
| OKM-1 | $muIgG_{2b}$ | CD11b | CR-3 |
| anti-Leu M5 | $muIgG_{2b}$ | CD11c | a chain (gp 150-95) |
| Anti-CR-1 | $muIgG_1$ | CD35 | C3b, CR-1 |
| anti-Leu M3 | $muIgG_{2b}$ | CD14 | Monocytes |

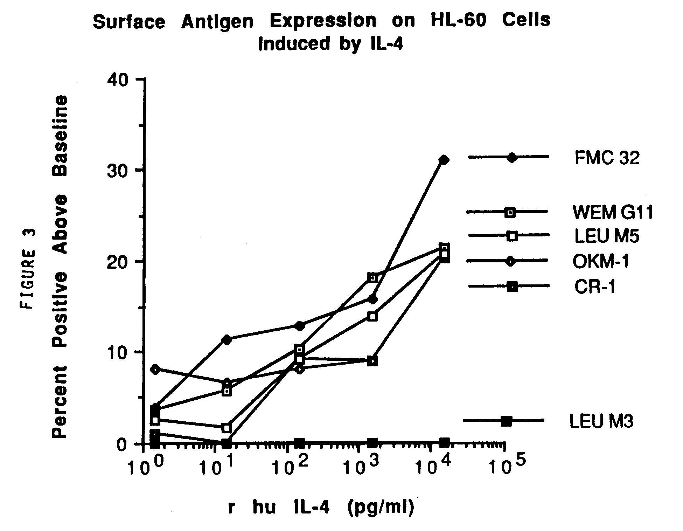

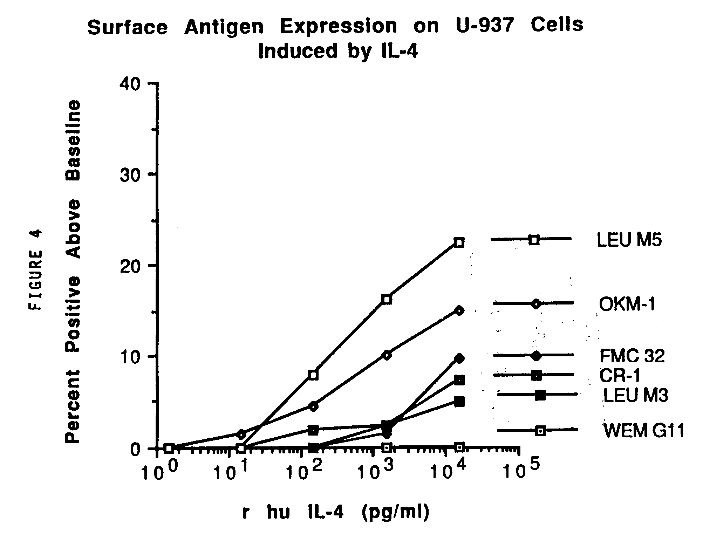

The results from the FACS Analysis above are shown in Figures 3 and 4.

**Claims**

1. Use of IL-4 in the manufacture of a pharmaceutical composition for increasing the numbers of neutrophils in a mammal.

2. Use of IL-4 in the manufacture of a pharmaceutical composition for treating myeloid or monocytoid leukaemia in a mammal.

3. Use according to Claim 1 wherein the mammal is a human.

4. Use according to Claim 1 or 3 in which the IL-4 is human IL-4.

5. Use according to Claim 1, 3 or 4 in which the IL-4 is administered in combination with at least one other white blood cell increasing agent.

6. Use according to Claim 5 in which the other white blood cell increasing agent is selected from granulocyte-macrophage colony stimulating factor, granulocyte colony stimulating factor, IL-1, IL-2, IL-3, IL-5, IL-6 or interferon alpha.

7. Use according to any of Claims 1, 3 or 4 in which the IL-4 is administered in combination with at least one chemotherapeutic agent.

8. Use according to Claim 7 in which the chemotherapeutic agent is selected from an alkylating agent, mitotic spindle poison or anti-tumor antibiotic.

**Patentansprüche**

1. Verwendung von IL-4 bei der Herstellung einer pharmazeutischen Zusammensetzung zur Erhöhung der Anzahl von Neutrophilen bei einem Säuger.

2. Verwendung von IL-4 bei der Herstellung einer pharmazeutischen Zusammensetzung zur Behandlung von Myelose oder Monocytenleukämie bei einem Säuger.

3. Verwendung gemäß Anspruch 1, wobei der Säuger ein Mensch ist.

4. Verwendung gemäß Anspruch 1 oder 3, wobei das IL-4 menschliches IL-4 ist.

5. Verwendung gemäß Anspruch 1, 3 oder 4, wobei das IL-4 in Kombination mit wenigstens einem anderen Mittel, das die Zahl weißer Blutkörperchen erhöht, verabreicht wird.

6. Verwendung gemäß Anspruch 5, wobei das andere Mittel, das die Zahl weißer Blutkörperchen erhöht, aus dem Granulocyten-Makrophagen-Kolonie-stimulierenden Faktor, dem Granulocyten-Kolonie-stimulierenden Faktor, IL-1, IL-2, IL-3, IL-5, IL-6 oder α-Interferon ausgewählt wird.

7. Verwendung gemäß einem der Ansprüche 1, 3 oder 4, wobei das IL-4 in Kombination mit wenigstens einem Chemotherapeutikum verabreicht wird.

8. Verwendung gemäß Anspruch 7, wobei das Chemotherapeutikum aus einem Alkylierungsmittel, einem Mitosespindelgift oder einem Antitumor-Antibiotikum ausgewählt wird.

**Revendications**

1. Utilisation de IL-4 dans la fabrication d'une composition pharmaceutique pour augmenter les nombres de neutrophiles chez un mammifère.

2. Utilisation de IL-4 dans la fabrication d'une composition pharmaceutique pour le traitement de la leucémie myéloïde ou monocytoïde chez un mammifère.

3. Utilisation selon la revendication 1, où le mammifère est un humain.

4. Utilisation selon la revendication 1 ou 3, où IL-4 est IL-4 humaine.

5. Utilisation selon la revendication 1, 3 ou 4, où IL-4 est administrée en association avec au moins un autre agent augmentant les globules blancs.

6. Utilisation selon la revendication 5, où l'autre agent augmentant les globules blancs est choisi parmi un facteur stimulant les colonies de granulocytes-macrophages, un facteur stimulant les colonies de granulocytes, IL-1, IL-2, IL-3, IL-5, IL-6 ou l'interféron alpha.

7. Utilisation selon l'une des revendications 1, 3 ou 4, où IL-4 est administrée en association avec au moins un agent chimiothérapeutique.

8. Utilisation selon la revendication 7, où l'agent chimiothérapeutique est choisi parmi un agent alkylant, un poison du fuseau mitotique ou un antibiotique antitumeur.

FIGURE 1

```
  1  H K C D I T L Q E I I K T L N S L T E Q K T L C T E L T V T
 31  D I F A A S K N T T E K E T F C R A A T V L R Q F Y S H H E
 61  K D T R C L G A T A Q Q F H R H K Q L I R F L K R L D R N L
 91  W G L A G L N S C P V K E A N Q S T L E N F L E R L K T I M
121  R E K Y S K C S S
```

FIGURE 2

FIGURE 3

EP 0 410 750 B1

FIGURE 4

Surface Antigen Expression on U-937 Cells Induced by IL-4

18